(19) Europäisches Patentamt / European Patent Office / Office européen des brevets

(11) **EP 4 058 075 B1**

(12) **EUROPEAN PATENT SPECIFICATION**

(45) Date of publication and mention
of the grant of the patent:
**20.08.2025 Bulletin 2025/34**

(21) Application number: **20803817.4**

(22) Date of filing: **09.11.2020**

(51) International Patent Classification (IPC):
*A61L 27/44* (2006.01)    *A61L 27/46* (2006.01)
*A61L 27/48* (2006.01)    *A61L 27/54* (2006.01)
*A61L 27/58* (2006.01)    *A61L 31/12* (2006.01)
*A61L 31/14* (2006.01)    *A61L 31/16* (2006.01)

(52) Cooperative Patent Classification (CPC):
(C-Sets available)
**A61L 27/54; A61L 27/446; A61L 27/46;
A61L 27/48; A61L 27/58; A61L 31/128;
A61L 31/129; A61L 31/148; A61L 31/16**    (Cont.)

(86) International application number:
**PCT/EP2020/081407**

(87) International publication number:
**WO 2021/094227 (20.05.2021 Gazette 2021/20)**

(54) **FIBER REINFORCED COMPOSITIONS AND METHODS OF MANUFACTURE FOR MEDICAL DEVICE APPLICATIONS**

FASERVERSTÄRKTE ZUSAMMENSETZUNGEN UND VERFAHREN ZUR HERSTELLUNG FÜR MEDIZINGERÄTEANWENDUNGEN

COMPOSITIONS RENFORCÉES PAR DES FIBRES ET PROCÉDÉS DE FABRICATION POUR DES APPLICATIONS DE DISPOSITIFS MÉDICAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.11.2019 US 201916685786**

(43) Date of publication of application:
**21.09.2022 Bulletin 2022/38**

(73) Proprietor: **Evonik Operations GmbH
45128 Essen (DE)**

(72) Inventors:
• **SANTIAGO-ANADON, Jose
Homewood, Alabama 35209 (US)**
• **DADSETAN, Mahrokh
Birmingham, Alabama 35211 (US)**
• **PRABHU, Balaji
Hoover, Alabama 35226 (US)**
• **LAWSON, Ryan
Birmingham, Alabama 35226 (US)**

(74) Representative: **Evonik Patent Association
c/o Evonik Industries AG
IP Management
Postcode 84/339
Rodenbacher Chaussee 4
63457 Hanau (DE)**

(56) References cited:
**EP-A1- 2 243 500        EP-A2- 1 980 279
WO-A1-2013/098481      US-A1- 2010 121 463
US-A1- 2018 297 239      US-B1- 6 709 995**

• **HAIBIN NING ET AL: "A review of Long fibre thermoplastic (LFT) composites", INTERNATIONAL MATERIALS REVIEWS, vol. 65, no. 3, 11 March 2019 (2019-03-11), US, pages 164 - 188, XP055771892, ISSN: 0950-6608, DOI: 10.1080/09506608.2019.1585004**

**(Cont. next page)**

(52) Cooperative Patent Classification (CPC): (Cont.)

C-Sets
**A61L 27/446, C08L 67/04;**
**A61L 27/46, C08L 67/04;**
**A61L 27/48, C08L 67/04;**
**A61L 31/128, C08L 67/04;**
**A61L 31/129, C08L 67/04**

**Description**

## FIELD OF THE INVENTION

The following invention relates to processing methods of making fiber reinforced polymeric compositions suitable for medical devices. Particularly compositions from polymeric fibers incorporated into a resorbable matrix, which can be injection molded. The fibers greatly enhance the mechanical properties of the composite. Also disclosed is the addition of inorganic additives to the matrix and/or the reinforcing fibers, which results in additional increase in mechanical properties while providing secondary effects such as osteoconductivity.

## BACKGROUND OF THE INVENTION

Tormala et. al. describes in US 4743257 and US 4968317 resorbable polymer compositions where the matrix and the reinforcing fiber are the same. Such self-reinforcement creates higher strength by drawing the material to produce highly aligned fibrils within the polymer thus increasing the mechanical properties of the material along the axis of alignment.

WO 90/12605 describes fiber reinforced bioerodible polymer compositions which include inorganic additives as well as oriented polymer fibers as a reinforcing component with the distinction that if the matrix is other than a poly(ortho ester), such as poly(lactic acid) the reinforcing additive must contain calcium-sodium metaphosphate, and when the reinforcement is other than calcium-sodium metaphosphate the matrix must include a poly(ortho ester).

WO 96/00592 describes a biodegradable reinforcement material that includes a pharmacological active ingredient with a biodegradable polymer layer, and a biodegradable reinforcing structure dispersed within the polymer layer. Such composition was described as having layers of reinforcement stacked with layers of polymer pressed together to incorporate the reinforcing layer into the polymer. A similar composition with layers of aligned fibers is described in US 20170246356A1.

In US 7541049, fiber reinforced formulations are described where a bioceramic, or bioactive particles of size between 60 to 150 microns are dispersed into a resorbable matrix, and a resorbable polymer fibers are incorporated into said composite matrix as well. However, their processing method requires compression molding of fiber and powder to form an article, which is not as scalable as other methods such as injection molding.

Although widely used in the medical field, the currently used resorbable polymers for implantable medical devices have for the most part limitations on their mechanical properties. Highly elastic and ductile materials such as poly(dioxanone) (PDO), poly(caprolactone) (PCL), and poly (trimethylene carbonate) (PTMC) can only achieve tensile strengths of 20 to 30 MPa, whereas high strength polymers such as poly(lactides) (PLA) and poly(glycolides) (PGA) are much stronger, but also highly brittle. These limitations have excluded these materials from a large segment of medical device applications where load bearing capacity is paramount. Their current use is mostly in sports medicine with devices, such as suture anchors, interference screws, as well as non-load bearing resorbable plating systems for cranio-maxillofacial applications. Still the promise of resorbable plating systems for internal fracture fixation is desirable, since the material would restore normal function to the bone after fixation compared to plating systems, which would require a second surgery to remove the metallic plates and screws. Upper extremity periarticular plating systems are among the first in line candidates for such materials, especially given the prevalence of fractures of the distal radius and proximal humerus. US2018/297239 A1 discloses a process for the production of a fiber-composite material where one or more fiber bundles may be gently expanded and subsequently impregnated with a melt.

The present invention describes a long fiber resorbable thermoplastic process of making compositions of materials, which are suitable for these kinds of load bearing applications, wherein fibers are incorporated into a resorbable polymer matrix through an impregnation process, which requires a polymer with low viscosity and with a melting temperature sufficiently lower than that of the fibers to allow the fibers to be fully impregnated or wetted by the polymer without affecting their strength or melting them.

Materials having a resorbable matrix with a resorbable synthetic fiber have been extensively described, most recently in US 7541049 and US 2017/0246356. A limiting factor for such compositions is the difference in melting temperatures of the matrix polymer and the reinforcing fiber, which make them unsuitable for injection molding. One of the most common examples of this are compositions of a PLA matrix reinforced with highly oriented PGA fibers. The melting point of PLA is approximately 180°, whereas the melting point of PGA is approximately 215°C measured by differential scanning calorimetry DSC. Such composition can easily be compression molded at 180°-185°C, however injection molding requires good flowability of the material to be able to pass through the mold gate and cavity, additionally the higher the fiber concentration the higher the need for good flowability of the matrix material. To injection mold these compositions, the material needs to be heated above 200°C. At this temperature, the fibers significantly soften, and lose most of their strength due to loss of molecule orientation, or flat out melt if the injection molding temperature exceeds the melting point of the PGA. It is because of this limitation that the field only focuses on compression molded compositions of a resorbable synthetic matrix and a resorbable synthetic fiber, but injection molding compositions do not exist in the field. To provide a fiber reinforced resorbable material that can be injection molded, companies are turning their focus to the use of non-synthetic fibers such as bioglass. Materials that incorporate these fibers can be processed with ease at high temperatures without the fibers weakening or melting. Bioglass can be made into strong fibers, but once implanted the fiber reinforced material loses strength rapidly due to the material's fast degradation and pH change under bodily fluid conditions. Bioglass fibers are also radiopaque, which limits the surgeon's ability to monitor the placement of implants under fluoroscopy in order to assess the bone fusion. The use of polymeric fibers however, do not have that restriction since they are radiolucent.

The disclosed compositions differ from the prior art in this regard in two key aspects, processing method and selection of matrix / fiber. The prior art describes their fiber impregnation methods as solvent mixing, melt mixing using a twin-screw extruder and powder mixing. These methods are either not scalable (due to no being cost effective), as is the case for solvent and powder mixing, or are ineffective due to their inability to produce material with sufficiently long fibers in the case of mixing the fibers with the matrix polymer inside a twin-screw extruder. The current invention also describes a method called long fiber resorbable thermoplastic (LFRT). The advantage of LFRT is its ability to incorporate fibers into a base polymer with improved impregnation while limiting the time that the fiber is exposed to heat, as well as maintaining tension on the fiber while processing. These two aspects prevent polymer fibers from melting or shrinking to the point that they lose their strength due to polymer chain relaxation. Having thoroughly wetted fibers with minimal loss in strength makes the process particularly suitable for making pre-pegs (pellets with fibers pre impregnated with matrix polymer), which can be injection molded. Poorly wetted fibers agglomerate during injection molding, and as a result, the material does not possess good flowability, which makes it difficult to injection mold, leading to injection molded parts with poor strength and quality.

## SUMMARY OF THE INVENTION

The scope of this invention is defined by the claims. Any "embodiment" or "example" which is disclosed in the description but is not covered by the claims should be considered as presented for illustrative purposes only. The present invention is directed to a process of making compositions that increase the mechanical properties of the base polymer by using reinforcing fibers dispersed into a resorbable matrix.

The matrix described is made from polycaprolactone, which resorb inside the body after implantation. The reinforcing fibers comprise poly(vinyl alcohol) or polyglycolide.

Additives can be incorporated into the matrix material or fibers to provide a secondary effect. These additives can be bioceramics or bioactive glass to produce an osteoconductive effect, antimicrobial particles such as silver, coloring agents, and radiopaque additives to make the implants visible under fluoroscopy. The incorporation of submicron sized additive particles into the matrix or into the fiber themselves result in an increase in tensile strength attributed to an increase in the interfacial bonding between the matrix and the fiber due to increased roughness, as well as increased heat transfer during molding between the fiber and the matrix. The disclosed compositions can be processed via both injection molding and compression molding. This is a key distinction between other compositions that rely on only compression molding methods due to the limited difference between the melting points of that of the fiber and the matrix.

## BRIEF DESCRIPTION OF THE DRAWINGS

The above and other objects, features and advantages of the present invention will be more clearly understood from the following detailed description taken in conjunction with the accompanying drawing, in which:

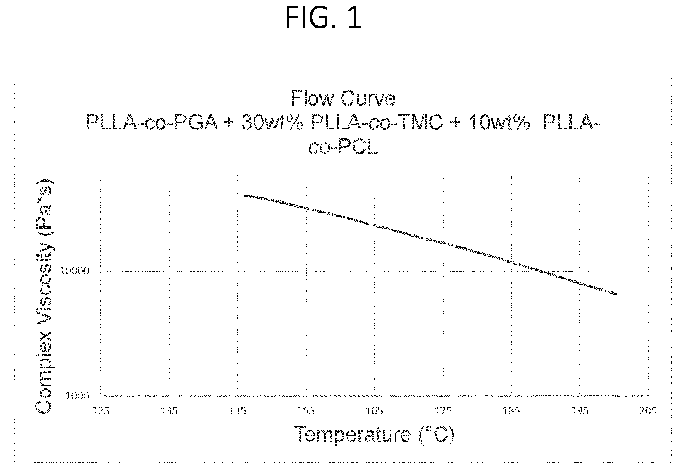

FIG. 1 depicts the rheology results that show that the base polymer would need a temperature of about 184°C to achieve a viscosity of 12000 Pa*s.

## DETAILED DESCRIPTION OF THE INVENTION

Definition of Terms

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an" and "the" include plural references unless the context clearly dictates otherwise. The present disclosure also contemplates other embodiments "comprising," "consisting of" and "consisting essentially of," the embodiments or elements presented herein, whether explicitly set forth or not.

The conjunctive term "or" includes any and all combinations of one or more listed elements associated by the conjunctive term. For example, the phrase "an apparatus comprising A or B" may refer to an apparatus including A where B is not present, an apparatus including B where A is not present, or an apparatus where both A and B are present. The phrases "at least one of A, B, ... and N" or "at least one of A, B, ... N, or combinations thereof" are defined in the broadest sense to mean one or more elements selected from the group comprising A, B, ... and N, that is to say, any combination of one or more of the elements A, B, ... or N including any one element alone or in combination with one or more of the other elements which may also include, in combination, additional elements not listed.

The modifier "about" used in connection with a quantity is inclusive of the stated value and has the meaning dictated by the context (for example, it includes at least the degree of error associated with the measurement of the particular quantity). The modifier "about" should also be considered as disclosing the range defined by the absolute values of the two endpoints. For example, the expression "from about 2 to about 4" also discloses the range "from 2 to 4." The term "about" may refer to plus or minus 10% of the indicated number. For example, "about 10%" may indicate a range of 9% to 11%, and "about 1" may mean from 0.9-1.1. Other meanings of "about" may be apparent from the context, such as rounding off, so, for example "about 1" may also mean from 0.5 to 1.4.

The term "wt. %" means weight percent.

The term "w/w" means weight per weight.

For the purposes of the present invention, the term "biodegradable" refers to polymers that dissolve or degrade in vivo within a period of time that is acceptable in a particular therapeutic situation. Such dissolved or degraded product may include a smaller chemical species. Degradation can result, for example, by enzymatic, chemical and/or physical processes. Biodegradation takes typically less than five years and usually less than one year after exposure to a physiological pH and temperature, such as a pH ranging from 6 to 9 and a temperature ranging from 22°C to 40°C.

For the purposes of the present invention, the term "radiopacity" means being visualized by x-ray.

For the purposes of the present invention, the term "tensile strength" refers to the maximum stress of a material to break under tension.

For the purposes of the present invention, the term "e-modulus" refers to the modulus of elasticity of the material under tension. It is the slope of the stress-strain curve of the material in the elastic region.

For the purposes of the present invention, the term "e-break" refers to the elongation at break. It is the ratio between the increase in length of a material subject to an axial load necessary for breaking the specimen and the initial length of the material.

For the purposes of the present invention, the term "injection molding" refers to the manufacturing method of producing parts where the melted plastic material is injected into a mold.

For the purposes of the present invention, the term "compression molding" refers to the manufacturing method of producing parts by placing the plastic material, sometimes preheated, in a mold, which is then subject to heat and pressure.

For the purposes of the present invention, the term "drawn fiber process" refers to the common practice of making polymeric fibers, where the fibers are melted, routed through a die, cooled off, and subsequently pulled by controlling their temperature and rate of pull to produce strong fibers with high molecular orientation.

For the purposes of the present invention, the term "thermoforming" refers to a manufacturing process where a plastic sheet is heated to soften the material to form it over a mold.

For the purposes of the present invention, the term "computer numerical control machining" refers to the common practice of forming a component or part from a stock shape by removing material using machining tools such as mills, drills, lathes where the movement of the tool is automated using a computer.

The present invention is directed to a process of making fiber reinforced compositions suitable for medical implantable devices having a resorbable matrix phase and one or more reinforcing phases, as disclosed in claim 1. In one embodiment, the fiber reinforced compositions contain additives incorporated into the matrix phase. In one embodiment, the fiber reinforced compositions contain additives incorporated into the fiber phase. In one embodiment, the fiber reinforced compositions contain additives in both the matrix phase and the fiber phase.

The present invention is directed to a process of making compositions of materials which are suitable for load bearing applications. Fibers are incorporated into a resorbable polymer matrix through an impregnation process. The impregnation process requires a polymer with low viscosity and with a melting temperature sufficiently lower than that of the fibers. This allows the fibers to be fully wetted by the polymer without affecting their strength or melting them. Materials ready to be post processed using molding methods, such as injection or compression molding are often referred to as "pre-pegs" alluding to the pre-impregnation process. In one embodiment, pre-impregnated forms can be pellets suitable for injection molding or compression molding. In one embodiment, pre-impregnated forms can be sheets that can be compression molded or thermoformed. In one embodiment, pre-impregnated forms can be rods and bars which can be machined.

Impregnated or wetted defines a measure of the fibers to be thoroughly coated by the matrix polymer. When there is good impregnation most of the fibers are coated by the polymer, which allows them to disperse better when injection molding. The result of this is an injection molded article that has uniform properties and better strength. In contrast, when there is bad impregnation the matrix polymer forms a "jacket" around the fibers without thoroughly wetting them. When these pre-pegs are injection molded into fibers, they don't disperse and instead tend to agglomerate.

The present invention uses a method called long fiber resorbable thermoplastic (LFRT). LFRT is a modification of the common long fiber thermoplastic (LFT) processing method. During LFT processing the matrix material is melted using a single or twin screw into a fiber impregnation die. The die pools the melted polymer into a vat and fiber is forced to go through the vat via a series of high pressure zones. Although the method works extremely well for some polymers especially resins and non-degradable thermoplastics, it presents serious challenges when processing materials highly susceptible to thermal degradation such as PLA in that the melted pool of polymer will degrade the material much more and with more variability. In contrast, the LFRT method relies on continuous flow of matrix material, and does not use a pool of material for the impregnation process. The polymer is melted and directed through a die, where it meets the fiber. The two phases are combined by pulling the fibers with the polymer through a flat portion of the die with rollers or high-pressure zones, which force the melt into the fiber. This way the polymer material is continually flowing through the die and no pooling occurs. One of the advantages of using this method is its versatility to produce fiber reinforced composites, which can be tape, strand or ribbons with continuous fibers suitable for compression molding sheets. If pelletized, upon injection molding the parts can be short or long fiber thermoplastics depending on the length of cut of the pellet.

The present invention uses polymers with low melting temperature comprising polycaprolactone at low molecular weight coupled with fibers with high melting points, comprising poly(vinyl alcohol) or polyglycolide.

The use of these materials allows for improved flowability at lower temperatures, which in turn improves the impregnation and injection moldability. Good impregnation requires a matrix polymer with a low enough viscosity, which can be adjusted by controlling the matrix polymer temperature. However, the matrix polymer processing temperature cannot be high enough as to melt the reinforcing fiber. Empirical data suggests that the processing temperature of the matrix polymer must not exceed the glass transition temperature of the reinforcing fibers plus 132°C ($T_g$ + 132°C).

$$T_P < T_g(Fiber) + 132°C$$

Different polymers have different flow properties and characteristics depending on their class (thermoplastics, thermosets, and elastomers), degree of crystallinity, molecular weight, moisture, etc., and the processing temperature described above will be largely influenced by those factors. Multiple methods can be used to define the processing temperature such as rheology, melt flow analysis, or DSC, which gives the melting temperature of the polymer (assuming it has one).

In one of the examples presented, the processing temperature is defined as the minimum temperature necessary such that the matrix polymer will have a complex viscosity of no higher than 12,000 Pa*s as measured by parallel plate rheology. This temperature would provide low enough viscosity such that the matrix polymer would flow through the LFRT extrusion head. Using this definition for the processing temperature yields a selection criterion for determining the processability of matrix / fiber combinations as follows:

$$T(Matrix\ Polymer)@12\ kPa \cdot s < T_g(Fiber) + 132°C$$

Table 1 depicted below provides examples of fiber matrix compositions applying the relationship stated above, but only polycaprolactone and poly(vinyl alcohol) or polyglycolide fibers are fiber matrix compositions falling within the scope of the claimed subject-matter. The complex viscosity of the base polymer is obtained via rheology methods.

### Table 1

| Matrix | | Temp. (°C) @ 12k Pa*s Complex Viscosity | | FIBER | | |
|---|---|---|---|---|---|---|
| | | | | PGA | PLLA | PVA |
| | | | | Tg (°C) | | |
| | | | | 38 | 60 | 80 |
| Matrix | PCL | | 60 | Yes | Yes | Yes |
| | PDO | | 131 | Yes | Yes | Yes |
| | PLCL | | 176 | No | Yes | Yes |
| | PLGA | | 195 | No | No | Yes |
| | PLLA | | 239 | No | No | No |
| | PLGA Blend | | 184 | No | Yes | Yes |

With the processing methods described above, a wide array of fibers can be impregnated with a resorbable matrix polymer, co-polymer, or blend, but only a resorbable matrix comprising polycaprolactone and fibers comprising polyglycolide or poly(vinyl alcohol) (PVA) are falling under the scope of the claimed subject-matter. In one embodiment, fibers are poly(vinyl alcohol) (PVA). PVA is highly biocompatible and widely used in pharmaceutical and tissue engineering applications. Fibers with very high tenacity can be manufactured from PVA and incorporated into a resorbable matrix.

In one embodiment, PVA is fully hydrolyzed and therefore non-soluble or partially hydrolyzed with varying degrees of solubility.

Some of the embodiments presented in this invention use additives to provide a secondary effect. In one embodiment, the additives are added to the matrix. In one embodiment, the additives are added to the fibers. In one embodiment, the additives are added to both the matrix and the fibers.

In one embodiment, the additives are inorganic additives, which include but are not limited to beta-tricalcium phosphate (β-TCP), hydroxyapatite (HA), and biphasic calcium phosphate (BCP). Inorganic additives can provide an osteoconductive effect.

The inclusion of additives to the matrix has been covered in US4743257 along with two fiber phases, one synthetic and one bioactive or bioceramics. The incorporation of the synthetic fibers in that work was to promote bonding between the bioceramics fiber phase and the matrix phase. Unlike the prior art, the supporting examples for this invention demonstrate that sub-micron sized particles of beta-tri-calcium phosphate improve the interfacial bonding between the matrix and the fiber by improving the surface roughness of such interface, which results in an increase in tensile strength of the composite material over the same material without the inorganic additive. Furthermore, the supporting examples demonstrate this strengthening effect when the additive is added to the matrix as well as to the fiber, that is synthetic drawn fibers made from a resorbable polymer composite containing inorganic sub-micron sized particles.

### EXAMPLES

The following examples are put forth to provide those of ordinary skill in the art with a complete disclosure and description of how the methods claimed herein are made and evaluated and are intended to be purely exemplary of the invention and are not intended to limit the scope of what the inventors regard as their invention, which is defined by the appended claims.

Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric. All tensile specimens in this disclosure follow ISO 527 testing protocol "Plastics - Determination of tensile properties" under the following conditions:

1. Test speed from 0% to 0.3% strain: 0.2mm/min

2. Test speed above 0.3% strain: 5mm/min

3. Determination of modulus of elasticity strain range: 0.05% to 0.025%

4. Injection Molded Specimen Type: ISO 527-2 5A

     a. Thickness: 2mm

     b. Width: 4mm

     c. Grip to grip distance: 50mm

     d. Length at narrow portion: 25mm

5. Compression Molded Specimen Type: ISO 527-2 1BA

     a. Thickness: 2mm

     b. Width: 5mm

     c. Grip to grip distance: 58mm

     d. Length at narrow portion: 25mm

EXAMPLE 1

PCL material (RESOMER® C209 commercially available from Evonik) was melted on a twin-screw extruder (commercially available from Thermo Scientific) and combined with a bundle of aligned continuous synthetic fibers introduced to the extrusion melt via a Long Fiber Thermoplastic Extrusion custom made die. One experiment used commercially available PVA fibers with a denier of 1.8dpf and a subsequent experiment used commercially available PGA fibers with a denier of 2.5dpf. Each of the extrudates, with PGA and PVA fibers, had 40 wt% incorporated fibers. The material was cut to 14mm length and injection molded to ISO 527-2 5A specimens. Injection molding temperature was 120°C. The resulting specimens were aged for two days at room temperature. Specimens were also manufactured this same way using only PCL material with no reinforcing fibers. Table 2 depicts the tensile strength, e-modulus, and e-break data of the specimens. Modulus of elasticity for the unreinforced specimens was 631 MPa compared to the PVA reinforced specimens' value of 5939 MPa and 3087MPa for PGA fiber reinforcement. Tensile Strength was 22.1 MPa for the PCL specimens, 79.7 MPa for PVA and 90.7 MPa for PGA reinforced specimens. Elongation at break was 277%, 11.3% and 13.8 for PCL, PCL with 40 wt% PVA fibers and PCL with 40 wt% PGA fibers respectively.

**Table 2**

| Composition | Tensile Strength (MPa) | E- Modulus (MPa) | e-break (%) |
|---|---|---|---|
| PCL | 22.1 | 631 | 277 |
| PCL + 40% PVA Fiber | 79.7 | 5939 | 11.3 |
| PCL + 40% PGA Fiber | 90.7 | 3087 | 13.8 |

EXAMPLE 2

The same setup, LFRT process and PGA fiber as EXAMPLE 1 was used, and the matrix material was changed to a poly(l-lactide-co-glycolide) (PLLA-co-PGA) ternary blend including 30wt% poly(L-lactide-co-trimethylene carbonate) (PLLA-co-TMC) and 15wt% poly(L-lactide-co-caprolactone) PLLA-co-PCL. Processing of these materials was not feasible due to the high viscosity of the matrix material. Processing at low temperature resulted in poorly impregnated fibers where the matrix polymer simply surrounded the fiber bundle without wetting it. Processing at high temperature resulted in the fiber bundle breaking during processing due to high temperature. Following selection criteria previously shown it can be easily corroborated that such combination would not be suitable for this process. The glass transition temperature of PGA is 38°C and adding 132°C to it gives us a maximum processing temperature of 170°C. The rheology results in FIG. 1 show that the base polymer would need a temperature of about 184°C to achieve a viscosity of 12000 Pa*s, which is far above the maximum processing temperature for the PGA fiber.

EXAMPLE 3

Compositions of fiber reinforced PCL (RESOMER® C209 commercially available from Evonik) were made by melting the matrix polymer using a twin-screw extruder and incorporating aligned continuous fibers into the polymer by means of a long fiber resorbable thermoplastic extrusion die. The inorganic additive was β-TCP with a particle size distribution D50 of 500 to 700 nanometers. The fibers used were PVA and PGA with deniers of 1.8 and 2.5 denier per filament (dpf) respectively.

Tensile specimens were made from these compositions using injection and compression molding methods. Injection molded specimens used ISO 527-2 5A specimen geometry, whereas compression molded specimens used ISO 527-2 1BA specimen geometry. The resulting specimens were aged for two days at room temperature. Table 3 depicts the tensile strength, e-modulus, and e-break data of the specimens.

Table 3

| Composition | Matrix | Fiber | Fiber Concentration % | Molding Method | Tensile Strength (MPa) | E-Modulus (GPa) | e-break (%) |
|---|---|---|---|---|---|---|---|
| | PCL | None | None | Injection | 22.1 | 0.6 | 277.0 |
| 3A | PCL | PVA | 30 | Injection | 85.0 | 5.6 | 5.5 |
| 3B | PCL + 5% β-TCP | PVA | 30 | Injection | 93.0 | 5.3 | 5.5 |
| 3A | PCL | PVA | 50 | Compression | 174.0 | 7.3 | 5.1 |
| 3B | PCL + 5% β-TCP | PVA | 50 | Compression | 334.0 | 10.4 | 9.5 |
| 3C | PCL | PGA | 60 | Compression | 166.0 | 6.1 | 9.7 |
| 3D | PCL + 5% β-TCP | PGA | 55 | Compression | 250.2 | 4.9 | 22.9 |

EXAMPLE 4

Compositions of PGA fiber reinforced PCL (RESOMER® C209 commercially available from Evonik) with β-TCP were made using the same methods described in example 3. The PGA fibers had a denier of 2.1 dpf and the β-TCP had a particle size distribution D50 of 500 to 700 nanometers. Tensile specimens were made from these compositions using injection and compression molding methods. Injection molded specimens used ISO 527-2 5A specimen geometry, whereas compression molded specimens used ISO 527-2 1BA specimen geometry. The resulting specimens were aged for two days at room temperature. Table 4 depicts the tensile strength, e-modulus, and e-break data of the specimens.

Table 4

| Composition | Matrix | Fiber | Fiber Concentration % | Molding Method | Tensile Strength (MPa) | E-Modulus (GPa) | e-break (%) |
|---|---|---|---|---|---|---|---|
| | PCL | None | None | Injection | 22.1 | 0.6 | 277.0 |
| 4A | PCL + 5% β-TCP | PGA | 38.9 | Injection | 66.16 | 3.0 | 16.9 |
| 4B | PCL + 10% β-TCP | PGA | 46.5 | Injection | 56.9 | 3.3 | 13.0 |
| 4A | PCL + 5% β-TCP | PGA | 59.4 | Compression | 264.7 | 6.3 | 22.9 |
| 4B | PCL + 10% β-TCP | PGA | 72.8 | Compression | 358.8 | 7.8 | 23.2 |

Reference EXAMPLE 5 Monofilament fibers with a diameter of 30 microns were made out compositions 5A and 5B shown in Table 5.

**Table 5**

| Composition | Description |
|---|---|
| 5A | PLLA (RESOMER® L210S commercially available from Evonik) with 15wt % PLLA-co-TMC 70:30 (RESOMER® LT706S) and 10wt% PLLA-co-PCL 70:30 (RESOMER® LC703S) |
| 5B | Composition A + 7.5% wt% β-tricalcium phosphate nanoparticles (500nm - 700nm) |

Fiber Compositions 5A and 5B were each separately combined with monofilament fibers of PDO (RESOMER® X206S) to a weight ratio of 70:30 (PDO:Fiber) and mixed using an air mixer. The mixtures were then fed into an injection molding machine and injection molded into a tensile specimen (ISO 527-2 5A) The specimens where molded at a temperature of 120°C. The resulting specimens were aged for two days at room temperature. Table 6 depicts the tensile strength, e-modulus, and e-break data of the specimens. The tensile strength of the fiber reinforced specimens with added inorganic ceramic inside the fiber exhibited an increase of 8.5% over the specimens without the additive (57.4 MPa to 62.3MPa).

**Table 6**

| Matrix | Fiber | Concentration (%) | Tensile Strength (MPa) | E-Modulus (GPa) | e-break (%) |
|---|---|---|---|---|---|
| PDO | None | None | 25.0 | 0.7 | 290.0 |
| PDO | Fiber 5A | 30.0 | 57.4 | 2.3 | 11.0 |
| PDO | Fiber 5B | 30.0 | 62.3 | 2.3 | 13.2 |

**Claims**

1. A long fiber resorbable thermoplastic process of making a composition comprising:

   a) a resorbable matrix comprising of polycaprolactone;
   b) one or more reinforcing fiber(s) impregnated into the resorbable matrix; wherein the reinforced fiber(s) comprise poly(vinyl alcohol) or polyglycolide and wherein the melting temperature of the resorbable matrix is lower than that of the fiber(s) and wherein the resorbable matrix has an inherent viscosity of not more than 2 dL/g and wherein the resorbable matrix and the reinforcing fiber(s) optionally contain additives, wherein the process comprises impregnating the materials in the reinforcing phase into the resorbable matrix phase by:

      a. melting the resorbable matrix phase using common methods such as extruder, reactor, or melt pump;
      b. routing the melted resorbable matrix phase into a die;
      c. routing the melted resorbable matrix phase inside the die to a flat strip area;
      d. feeding fiber bundles into the flat strip area where the bundles are incorporated into the melted resorbable matrix phase;
      e. pulling the fiber bundles and the melted resorbable matrix phase through the die over high-pressure zones indie the die, such as narrow sections or rollers;
      f. forcing the melted resorbable matrix phase into the fiber bundles by wetting the fiber bundles, thereby forming a pre-impregnated material coming out of the die; and
      g. collecting the pre-impregnated material that is formed into a flat strand or a circular strand; and
      h. optionally pelletizing the material for injection molding.

2. The process of claim 1, wherein the additives comprise:

   a) inorganic additives; and/or
   b) calcium phosphate salts containing a dopant; and/or
   c) radiopaque materials; and/or
   d) coloring agents; and/or
   e) biologics.

**3.** The process of claim 2, wherein

    i) the inorganic additives comprise apatites or calcium phosphates; or
    ii) the inorganic additives comprise apatites, wherein the apatites comprise hydroxyapatites (HA); or
    iii) wherein the calcium phosphates comprise β-tricalcium phosphate (β-TCP), or biphasic calcium phosphate (BCP); or
    iv) wherein the dopant comprises Fluorine (F), Sulphur (S), Boron (B), Strontium (Sr), Magnesium (Mg), Silver (Ag), Barium (Ba), Zinc (Zn), Sodium (Na), Potassium (K), Aluminium (Al), Titanium (Ti), Silicon (Si), or Copper (Cu); or
    v) wherein the radiopaque materials comprise barium sulfate, bismuth compounds, tantalum, or tungsten; or
    vi) wherein the coloring agents comprise monosodium salt of 2-[(9,10-dihydro-4-hydroxy -9,10-dioxo-1-anthracenyl) amino]-5-methyl-benzenesulfonic acid (D&C Violet NO. 2), D&C Blue NO. 6, or D&C Green NO. 6; or
    vii) wherein the biologics comprise antibacterial agent, proteins, peptides, growth factors, or antibiotics.

**4.** The process of any of claims 1 to 3, wherein the reinforcing fibers comprises multifilament or monofilament fibers which can be aligned continuous, chopped, woven or braided into strips, ribbons, strands, and other forms.

**5.** The process of any of claims 1 to 4, wherein the inclusion of an inorganic additive into the matrix, the fiber or both, results in increased tensile strength compared to compositions without an inorganic additive.

**6.** The process of any of claims 1 to 5, wherein the average particle size of the additive is less than 50 microns, preferably less than 1 micron.

**7.** The process of any of claims 1 to 6, wherein the matrix phase polymer material is between 30 wt% to 90 wt%, preferably 30 wt% to 89 wt%, the reinforcing phase fibers is between 10 wt% to 70 wt%, preferably 10 wt% to 69 wt%, by weight of the composition, and the additive(s) is between 1 wt% to 30 wt% by weight of the composition.

**8.** The process of any of claims 1 to 7, wherein the composition

    i) comprises a bioceramic additive having an average particle size of less than 50 microns, and preferably less than 1 micron; and/or
    ii) is formed into a semi-finished or finished medical device article; and/or
    iii) is processed into pellets, filaments, rods, or sheets; and/or
    iv) is processed by drawing into tubes or films; and/or
    v) is used to form an article, and wherein the article is annealed; and/or
    vi) is processed into pellets, wherein the pellets are injection molded or compression molded into articles; and/or
    vii) is processed into sheets, wherein the sheets are formed into articles by means of thermoforming; and/or
    viii) is processed into sheets or rods, wherein the sheets and rods are milled into articles using computer numerical control machining; and/or
    ix) is formed into an injection moldable specimen; and wherein the tensile strength of the injection moldable specimen is more than 2 folds of the matrix material.

**9.** The process of any of claims 1 to 8, wherein the concentration of the additive is 1 to 30 wt% by weight of the composition and preferably 1 to 5 wt% by weight of the composition.

**10.** The process of any of claims 1 to 9, wherein the length of the fibers is no more than 20mm and wherein the diameter of the fibers is no more than 15 microns.

**11.** The process of any of claims 1 to 10, wherein the composition is an injection moldable fiber reinforced material, wherein the resorbable matrix and the reinforcing fiber(s) comprises polymeric materials; wherein the polymeric materials are being selected in such a way that the processing temperature of the matrix polymer is less than the glass transition temperature of the fiber(s) plus 132°C.

**Patentansprüche**

**1.** Verfahren für resorbierbaren Thermoplast mit langen Fasern zur Herstellung einer Zusammensetzung, umfassend:

a) eine resorbierbare Matrix umfassend Polycaprolacton;

b) eine oder mehrere Verstärkungsfaser(n), die in die resorbierbare Matrix imprägniert sind; wobei die Verstärkungsfaser(n) Poly(vinylalkohol) oder Polyglycolid umfassen und wobei die Schmelztemperatur der resorbierbaren Matrix niedriger als jene der Faser(n) ist und wobei die resorbierbare Matrix eine inhärente Viskosität von nicht mehr als 2 dl/g aufweist und wobei die resorbierbare Matrix und die Verstärkungsfaser(n) gegebenenfalls Zusatzstoffe enthalten, wobei das Verfahren Imprägnieren der Materialien in der Verstärkungsphase in die resorbierbare Phase umfasst durch:

a. Schmelzen der resorbierbaren Matrixphase unter Verwendung gängiger Verfahren, wie z.B. Extruder, Reaktor oder Schmelzpumpe;

b. Leiten der geschmolzenen resorbierbaren Matrixphase in ein Düsenwerkzeug;

c. Leiten der geschmolzenen resorbierbaren Matrixphase innerhalb des Düsenwerkzeugs zu einem Flachbandbereich;

d. Zuführen von Faserbündeln in den Flachbandbereich, wo die Bündel in die geschmolzene resorbierbare Matrixphase einverleibt werden;

e. Ziehen der Faserbündel und der geschmolzenen resorbierbaren Matrixphase durch das Düsenwerkzeug über Hochdruckzonen in dem Düsenwerkzeug, wie z.B. enge Abschnitte oder Walzen;

f. Drücken der geschmolzenen resorbierbaren Matrixphase in die Faserbündel durch Benetzen der Faserbündel, um ein vorimprägniertes Material zu bilden, das aus dem Düsenwerkzeug austritt; und

g. Sammeln des vorimprägnierten Materials, das zu einem flachen Strang oder einem kreisförmigen Strang geformt ist; und

h. gegebenenfalls Pelletieren des Materials zum Spritzguss.

2. Verfahren nach Anspruch 1, wobei die Zusatzstoffe umfassen:

a) anorganische Zusatzstoffe; und/oder

b) Calciumphosphatsalze, die einen Dotierstoff enthalten; und/oder

c) strahlendichte Materialien; und/oder

d) Farbmittel und/oder

e) biologische Stoffe.

3. Verfahren nach Anspruch 2, wobei

i) die anorganischen Zusatzstoffe Apatite oder Calciumphosphate umfassen; oder

ii) die anorganischen Zusatzstoffe Apatite umfassen, wobei die Apatite Hydroxylapatite (HA) umfassen; oder

iii) wobei die Calciumphosphate β-Tricalciumphosphat (β-TCP) oder zweiphasiges Calciumphosphat (BCP) umfassen; oder

iv) wobei der Dotierstoff Fluor (F), Schwefel (S), Bor (B), Strontium (Sr), Magnesium (Mg), Silber (Ag), Barium (Ba), Zink (Zn), Natrium (Na), Kalium (K), Aluminium (Al), Titan (Ti), Silicium (Si) oder Kupfer (Cu) umfasst; oder

v) wobei die strahlendichten Materialien Bariumsulfat, Bismutverbindungen, Tantal oder Wolfram umfassen; oder

vi) wobei die Farbmittel Mononatriumsalz von 2-[(9,10-Dihydro-4-hydroxy-9,10-dioxo-1-anthracenyl)amino]-5-methylbenzolsulfonsäure (D&C Violet NO 2), D&C BLUE NO. 6 oder D&C Green NO. 6 umfassen; oder

vii) wobei die biologischen Stoffe antibakterielles Mittel, Proteine, Peptide, Wachstumsfaktoren oder Antibiotika umfassen.

4. Verfahren nach einem der Ansprüche 1 bis 3, wobei die Verstärkungsfasern Multifilament- oder Monofilamentfasern umfassen, die kontinuierlich ausgerichtet, geschnitten, gewebt oder zu Streifen, Bändern, Strängen und anderen Formen geflochten sein können.

5. Verfahren nach einem der Ansprüche 1 bis 4, wobei die Aufnahme eines anorganischen Zusatzstoffs in die Matrix, die Faser oder beide zu einer erhöhten Zugfestigkeit im Vergleich zu Zusammensetzungen ohne einen anorganischen Zusatzstoff führt.

6. Verfahren nach einem der Ansprüche 1 bis 5, wobei die mittlere Partikelgröße des Zusatzstoffs weniger als 50 Mikrometer, vorzugsweise weniger als 1 Mikrometer, beträgt.

7. Verfahren nach einem der Ansprüche 1 bis 6, wobei das Matrixphasen-Polymermaterial zwischen 30 Gew.-% und 90 Gew.-%, vorzugsweise 30 Gew.-% bis 89 Gew.-% beträgt, die Verstärkungsphasenfasern zwischen 10 Gew.-% und 70 Gew.-%, vorzugsweise 10 Gew.-% bis 69 Gew.-%, der Zusammensetzung betragen und der oder die Zusatzstoffe zwischen 1 Gew.-% bis 30 Gew.-% der Zusammensetzung betragen.

8. Verfahren nach einem der Ansprüche 1 bis 7, wobei die Zusammensetzung

   i) einen biokeramischen Zusatzstoff mit einer mittleren Partikelgröße von weniger als 50 Mikrometer und vorzugsweise weniger als 1 Mikrometer umfasst; und/oder
   ii) zu einem halbfertigen oder fertigen Medizinvorrichtungsgegenstand geformt wird; und/oder
   iii) zu Pellets, Filamenten, Stäben oder Folien verarbeitet wird; und/oder
   iv) durch Ziehen zu Schläuchen oder Folien verarbeitet wird; und/oder
   v) zum Bilden eines Gegenstands verwendet wird und wobei der Gegenstand getempert wird; und/oder
   vi) zu Pellets verarbeitet wird, wobei die Pellets zu Gegenständen spritzgegossen oder verpresst werden; und/oder
   vii) zu Platten verarbeitet wird, wobei die Platten durch Thermoformen zu Gegenständen geformt werden; und/oder
   viii) zu Platten oder Stäben verarbeitet wird, wobei die Platten und Stäbe durch Bearbeitung mit numerischer Computersteuerung zu Gegenständen gefräst werden; und/oder
   ix) zu einem spritzgießbaren Stück geformt wird; und wobei die Zugfestigkeit des spritzgießbaren Stücks mehr als 2-mal jener des Matrixmaterials beträgt.

9. Verfahren nach einem der Ansprüche 1 bis 8, wobei die Konzentration des Zusatzstoffs 1 bis 30 Gew.-% der Zusammensetzung und vorzugsweise 1 bis 5 Gew.-% der Zusammensetzung beträgt.

10. Verfahren nach einem der Ansprüche 1 bis 9, wobei die Länge der Fasern nicht mehr als 20 mm beträgt und wobei der Durchmesser der Fasern nicht mehr als 15 Mikrometer beträgt.

11. Verfahren nach einem der Ansprüche 1 bis 10, wobei die Zusammensetzung ein spritzgießbares faserverstärktes Material ist, wobei die resorbierbare Matrix und die Verstärkungsfaser(n) Polymermaterialien umfassen; wobei die Polymermaterialien so ausgewählt sind, dass die Verarbeitungstemperatur des Matrixpolymers niedriger als die Glasübergangstemperatur der Faser(n) plus 132 °C ist.

**Revendications**

1. Procédé thermoplastique résorbable à fibres longues de préparation d'une composition comprenant :

   a) une matrice résorbable comprenant une polycaprolactone ;
   b) une ou plusieurs fibres de renforcement imprégnées dans la matrice résorbable ; dans lequel la ou les fibres renforcées comprennent un poly(alcool vinylique) ou un polyglycolide et dans lequel la température de fusion de la matrice résorbable est inférieure à celle de la ou des fibres et dans lequel la matrice résorbable a une viscosité inhérente non supérieure à 2 dL/g et dans lequel la matrice résorbable et la ou les fibres de renforcement contiennent éventuellement des additifs, dans laquelle le procédé comprend l'imprégnation des matériaux dans la phase de renforcement dans la phase de la matrice résorbable par :

      a. fusion de la phase de matrice résorbable à l'aide de procédés courants tels qu'une extrudeuse, un réacteur ou une pompe de fusion ;
      b. acheminement de la phase de matrice résorbable fondue dans une filière ;
      c. acheminement de la phase de matrice résorbable fondue à l'intérieur de la filière vers une zone de bande plate ;
      d. alimentation des faisceaux de fibres dans la zone de la bande plate où les faisceaux sont incorporés dans la phase de matrice résorbable fondue ;
      e. tirage des faisceaux de fibres et la phase de matrice résorbable fondue à travers la filière sur des zones à haute pression dans la filière, telles que des sections ou des rouleaux étroit(e)s ;
      f. le fait de forcer la phase de matrice résorbable fondue dans les faisceaux de fibres en mouillant les faisceaux de fibres, formant ainsi un matériau pré-imprégné sortant de la filière ; et
      g. collecte du matériau pré-imprégné qui est formé en un brin plat ou un brin circulaire ; et
      h. éventuellement mise en pastilles du matériau pour un moulage par injection.

**2.** Procédé selon la revendication 1, dans lequel les additifs comprennent :

a) des additifs inorganiques ; et/ou
b) des sels de phosphate de calcium contenant un dopant ; et/ou
c) des matériaux radio-opaques ; et/ou
d) des agents colorants ; et/ou
e) des agents biologiques.

**3.** Procédé selon la revendication 2, dans lequel

i) les additifs inorganiques comprennent des apatites ou des phosphates de calcium ; ou
ii) les additifs inorganiques comprennent des apatites, les apatites comprenant des hydroxyapatites (HA) ; ou
iii) dans lequel les phosphates de calcium comprennent du phosphate β-tricalcique (β-TCP) ou du phosphate de calcium biphasique (BCP) ; ou
iv) dans lequel le dopant comprend Fluor (F), Soufre (S), Bore (B), Strontium (Sr), Magnésium (Mg), Argent (Ag), Baryum (Ba), Zinc (Zn), Sodium (Na), Potassium (K), Aluminium (Al), Titane (Ti), Silicium (Si) ou Cuivre (Cu) ; ou
v) dans lequel les matériaux radio-opaques comprennent du sulfate de baryum, des composés de bismuth, du tantale ou du tungstène ; ou
vi) dans lequel les agents colorants comprennent le sel monosodique de l'acide 2-[(9,10-dihydro-4-hydroxy-9,10-dioxo-1-anthracényl)amino]-5-méthyl-benzènesulfonique (D&C Violet n° 2), D&C Blue n° 6, ou N&C Green n° 6 ; ou
vii) dans lequel les agents biologiques comprennent un agent antibactérien, des protéines, des peptides, des facteurs de croissance ou des antibiotiques.

**4.** Procédé selon l'une quelconque des revendications 1 à 3, dans lequel les fibres de renforcement comprennent des fibres multifilamentaires ou monofilamentaires qui peuvent être alignées de manière continue, coupées, tissées ou tressées en bandes, rubans, brins et autres formes.

**5.** Procédé selon l'une quelconque des revendications 1 à 4, dans lequel l'inclusion d'un additif inorganique dans la matrice, la fibre ou les deux, entraîne une résistance à la traction accrue par rapport à des compositions sans additif inorganique.

**6.** Procédé selon l'une quelconque des revendications 1 à 5, dans lequel la taille moyenne de particule de l'additif est inférieure à 50 microns, de préférence inférieure à 1 micron.

**7.** Procédé selon l'une quelconque des revendications 1 à 6, dans lequel le matériau de polymère de la phase de matrice est présent entre 30 % en poids et 90 % en poids, de préférence entre 30 % en poids et 89 % en poids, les fibres de la phase de renforcement sont présentes entre 10 % en poids et 70 % en poids, de préférence entre 10 % en poids et 69 % en poids de la composition, et l'additif ou les additifs sont présents entre 1 % en poids et 30 % en poids de la composition.

**8.** Procédé selon l'une quelconque des revendications 1 à 7, dans lequel la composition

i) comprend un additif de biocéramique ayant une taille moyenne de particule inférieure à 50 microns, et de préférence inférieure à 1 micron ; et/ou
ii) est mise en forme en un article de dispositif médical semi-fini ou fini ; et/ou
iii) est transformée en pastilles, filaments, tiges ou feuilles ; et/ou
iv) est traitée par étirage en tubes ou en films ; et/ou
v) est utilisée pour mettre en forme un article, et dans lequel l'article est recuit ; et/ou
vi) est transformée en pastilles, les pastilles étant moulées par injection ou moulées par compression en articles ; et/ou
vii) est transformée en feuilles, les feuilles étant transformées en articles au moyen d'un thermoformage ; et/ou
viii) est transformée en feuilles ou en tiges, les feuilles et tiges étant broyées en articles par usinage à commande numérique par ordinateur ; et/ou
ix) est mise en forme en une éprouvette moulable par injection ; et dans lequel la résistance à la traction de l'éprouvette moulable par injection est supérieure à deux fois celle du matériau de matrice.

**9.** Procédé selon l'une quelconque des revendications 1 à 8, dans lequel la concentration de l'additif est de 1 à 30 % en poids de la composition et de préférence de 1 à 5 % en poids de la composition.

10. Procédé selon l'une quelconque des revendications 1 à 9, dans lequel la longueur des fibres n'est pas supérieure à 20 mm et dans lequel le diamètre des fibres n'est pas supérieur à 15 microns.

11. Procédé selon l'une quelconque des revendications 1 à 10, dans lequel la composition est un matériau renforcé par des fibres moulable par injection, dans lequel la matrice résorbable et la ou les fibres de renforcement comprennent des matériaux de polymère ; dans lequel les matériaux de polymère sont choisis de telle sorte que la température de traitement du polymère de matrice est inférieure à la température de transition vitreuse de la ou des fibres plus 132 °C.

# FIG. 1

Flow Curve
PLLA-co-PGA + 30wt% PLLA-*co*-TMC + 10wt%  PLLA-*co*-PCL

## REFERENCES CITED IN THE DESCRIPTION

**Patent documents cited in the description**

- US 4743257 A
- US 4968317 A
- WO 9012605 A
- WO 9600592 A
- US 20170246356 A1
- US 7541049 B
- US 2018297239 A1
- US 20170246356 A